# EUROPEAN PATENT APPLICATION

(11) **EP 4 666 987 A1**
(43) Date of publication of application: **24.12.2025**
(21) Application number: 24020205.1
(22) Date of filing: 18.06.2024
(51) Int. Cl.: A61F 2/24, A61F 2/06, A61F 2/07, A61F 2/95

(54) **A TRANSCATHETER ENDOLUMINAL PROSTHESIS, A DELIVERY SYSTEM CONTAINING IT AND A METHOD OF IMPLANTATION OF THE PROSTHESIS**

(71) Applicant: TT3A S.r.l., 20124 Milan (IT)
(72) Inventor: HURKALA, Leszek, 03-126 Warsaw (PL); GWIAZDOWSKA-NOWOTKA, Beata, 01-002 Warsaw (PL); SELMI, Matteo, 20049 Settala (Milan) (IT)
(74) Representative: Orlinska, Dorota Irena

(57) **Abstract**

The subject of the invention is a self-expanding, covered at least partially by a coating (16), endoluminal prosthesis, comprising:

- a stent-valve part (1"), containing a metal frame (10) and an aortic valve (14) secured inside it,

- a stent-graft part (1'), containing a metal frame (8, 8'),

- an intermediate part (4), joining the stent-valve part (1") with the stent-graft part (1') and having less stiffness than the stent-valve part (1") and the stent-graft part (1'),

wherein the stent graft part (1') comprises a tapered section (13) narrowing inwardly towards the intermediate part (4), the tapered section (13) is equipped with at least one branch (5', 5") covered by a coating (16).

## Description

### BACKGROUND OF THE INVENTION

The subject of the invention is a transcatheter endoluminal prosthesis, a delivery system containing the defined prosthesis, and a method of implantation of the prosthesis in the patient's body using a specified delivery system. The disclosed subject matter relates to a prosthesis and delivery system for treating endovascular irregularities within the aorta and especially for treatment of an ascending aorta aneurysm and ascending aorta dissection.

Thoracic aortic diseases including aneurysms and dissections of the thoracic aorta are a major cause of morbidity and mortality, especially in patients who have multiple comorbidities, hemodynamic instability or malperfusion, and end-organ dysfunction.

Aortic aneurysms are a disease typically manifested by an expansion of the aorta vessel wall. Aneurysms affect the ability of the vessel lumen to conduct blood. An endovascular standard treatment for repairing an aneurysm is to implant the replacement prosthesis into the damaged part of the vessel.

Aortic dissection occurs when the inner layer of the aorta's artery wall splits open (dissects). When the layers of the aortic wall split open (separate from one another), it creates a false lumen through which pulsatile blood flow can access the inner layers that compose the arterial wall. The flow of blood through this false lumen, and the resulting increase in pressure, can exacerbate the dissection and cause further tearing.

The standard surgical approach in patients with ascending aortic aneurysm or dissection involving the aortic root and associated with aortic valve disease is open heart surgery including the replacement of the aortic valve and ascending aorta. These procedures carry high risks, especially in older patients.

There thus remains a need for a safe and effective, minimally invasive percutaneous transcatheter (or transluminal) delivery of replacement cardiac prosthesis to solve the problems presented above.

The devices and methods disclosed herein can be used expediently via a catheter-based approach on a beating heart, avoiding potential morbidities associated with an open heart procedure.

Devices and delivery systems for treating the ascending aorta aneurysm are generally known.

US 2015157477 A1 discloses an intraluminal vascular prosthesis assembly for endovascular aortic repair. The prosthesis assembly has a hollow cylindrical body with a first end and a second end, comprising: at its first end, a first vascular prosthesis portion, and at its second end, a second vascular prosthesis portion, which has only a prosthesis material, wherein the vascular prosthesis assembly has a stent portion which is provided between the first vascular prosthesis portion and the second vascular prosthesis portion. The first vascular prosthesis portion and the second vascular prosthesis portion are designed for anchoring the vascular prosthesis assembly in the aorta. The prosthesis assembly includes a pair of conduits, each conduit is sized to receive a catheter or stent for coronary blood flow.

US 2014316513 A1 presents a medical device, for use within the aorta, including a valve member with an elongate tubular portion with at least one aperture disposed in a sidewall at a location between the proximal and distal ends, and a plurality of leaflets; a first endovascular graft coupled to the valve member; and a second endovascular graft coupled to the first endovascular graft; and an expandable anchor member coupled to the second endovascular graft.

WO2021231501 A1 discloses an endoluminal prosthesis, which includes a stent-graft and a temporary aortic valve, typically combined in an integrated assembly suitable for endoluminal placement in a patient's ascending aorta, aortic root, and aortic valve. The stent-graft has a base end, the temporary aortic valve assembly is attached to the base end of the stent-graft and comprises a scaffold configured to be anchored in the patient's aortic annulus and valve leaflets configured to function temporarily after the endoluminal prosthesis has been implanted. At least one fenestration suitable for receiving a guidewire and/or a coronary stent graft is located near a junction between the base end of the stent graft and the temporary aortic valve, wherein said at least one fenestration is disposed on the endoluminal prosthesis to be aligned with one of the patient's coronary ostia after the endoluminal prosthesis has been implanted.

### SUMMARY OF THE INVENTION

The subject of the invention is the self-expanding, covered at least partially by a coating, endoluminal prosthesis, comprising:
- as a distal portion, a stent-valve part, containing a metal frame and an aortic valve secured inside it, the metal frame is provided, with distal locking means, evenly distributed around the perimeter of the prosthesis, at its distal edge,
- as a proximal portion, a stent-graft part, containing a metal frame provided, at its proximal edge, with proximal locking means, evenly distributed around the perimeter of the prosthesis,
- an intermediate part, joining the stent-valve part with the stent-graft part and having less stiffness than the stent-valve part and the stent-graft part, the intermediate part contains either a coating alone, or, in addition, also metal connectors linking the metal frame of the stent-valve part with the metal frame of the stent-graft part,
wherein the stent graft part comprises, from a proximal side, a cylindrical section passing into a tapered section narrowing inwardly towards the intermediate part, the tapered section is equipped with at least one branch covered by the coating, and wherein the distal locking means and the proximal locking means are not covered by the coating .

In the preferred embodiment of the invention, the proximal locking means, present in the self-expanding prosthesis, have the form of peaks of a proximal row of wires and the distal locking means, present in the self-expanding prosthesis, are in the form of eyelets, hooks or loops.

In the preferred embodiment of the invention, the coating covers entire prosthesis and only the distal locking means and the proximal locking means remains uncovered by the coating.

In the preferred embodiment of the invention, the branch or branches is/are directed at an acute γ angle, with respect to the longitudinal X axis of the prosthesis, toward the stent valve part, and the angle γ range from 5° to 60°. Additionally, the branch or branches is/are attached to the tapered section through a bellow-shaped intermediate part.

The subject of the invention is also a delivery system comprising the defined prosthesis.

In the preferred embodiment of the invention, the delivery system comprises:
- an inner tube, extending along a longitudinal Y axis of the delivery system, from an atraumatic distal tip to a handle;
- a metal tube, slide-fitted on the inner tube, and extending from the internal space of the distal tip to a handle;
- an outer tubular catheter shaft slide-fitted on the metal tube, the catheter shaft is brought out from the handle and is terminated by a capsule, containing inside the radially compressed on the metal tube prosthesis;
- the capsule has closed position when is closed by the tip and has open position when the capsule and the tip are displaced relative to each other;
wherein the distal part of the metal tube is equipped with receiving means on which the distal locking means of prosthesis are removably embedded and blocked in the inner space of the tip and the delivery system comprises also removable holding means retaining the proximal locking means of the prosthesis, so that when the capsule is in its fully open position, the prosthesis is still maintained partially compressed, holding its crimping length.

In the preferred embodiment of the invention, the delivery system comprises preloaded, guided through the branch or branches of the prosthesis, at least one lateral guide wire routed in the proximal part of the delivery system along the internal channel of the microcatheter and of the guiding catheter.

In the preferred embodiment of the invention, on the metal tube a rotating paddle is permanently mounted in the section where the stent-graft part of the prosthesis is placed.

Additionally, in the delivery system, preferably, the receiving means are in the form of pins mounted on a barrel and the holding means are in the form of locking loops of locking wires.

The subject of the invention is also a method of delivering the endoluminal prosthesis defined above, to the patient's ascending aorta and aortic valve, using the delivery set comprising an introducer and a delivery system containing the endoluminal self-expanding prosthesis, the method comprising:
a) inserting the introducer into the blood vessel;
b) inserting a main guide wire into the blood vessel such that it extends through the blood vessel into the ascending aorta and aortic valve;
c) moving forward, over the main guide wire, preferably using the steering wire(-s) when crossing the aorta, and positioning up to above the level of the coronary arteries the compressed, deployable, locked from the proximal side and from the distal side, prosthesis, located inside the constricting capsule of the delivery system;
d) retrieving the constricting capsule up to the partially released configuration;
e) rotating the prosthesis to get alignment at least between one prosthesis branch and one coronary ostium;
f) introducing the at least one lateral guide wire, preloaded in the delivery system by positioning it/them in the branch or branches of the prosthesis, into the coronary artery (-ies), using the guiding catheter(-s), or optionally also the microcatheter(-s), as support for coronary(-ies) cannulation;
g) introducing, over the lateral guide wire(-s), the coronary stent graft system(-s) carrying coronary stent graft(-s), into the coronary artery(-ies);
h) moving forward the inner part of the delivery system comprising the inner tube and a metal tube with the prosthesis, over the main guide wire and preferably over the at least one guiding catheter up to the aortic valve, where the stent-valve part of the prosthesis will align with the proper location with respect to the native aortic valve;
i) unlocking the distal side of the prosthesis releasing its stent-valve part which anchors in the tissue surrounding the native valve;
j) unlocking the proximal side of the prosthesis releasing its stent-graft part;
k) depositing, after full deployment of the prosthesis, the coronary stent-graft(-s), provided by the coronary stent graft system(-s), in the left and/or right coronary arteries, wherein the expanded stent-graft(-s) are connected to the branch(-es) of the prosthesis by overlapping;
l) retracting the coronary stent graft system(-s), guiding catheter(-s) and the lateral guide wire(-s), the delivery system, and finally also the main guide wire,
wherein during the stages from (b) to (h) a positioning of the prosthesis is carried out, by its rotation, around its longitudinal axis, together with the metal tube, on which it is removably mounted, and wherein during the stages from (d) to (g) the delivery system is approximately still in the same position where the distal tip is positioned above the level of the coronary arteries.

Preferably, in stage (h) unlocking the distal side of the prosthesis and releasing its stent-valve part is performed according to the on/off mode, and unlocking the proximal side of the prosthesis and releasing its stent-graft part in stage (i) is performed gradually.

### BRIEF DESCRIPTION OF THE DRAWINGS

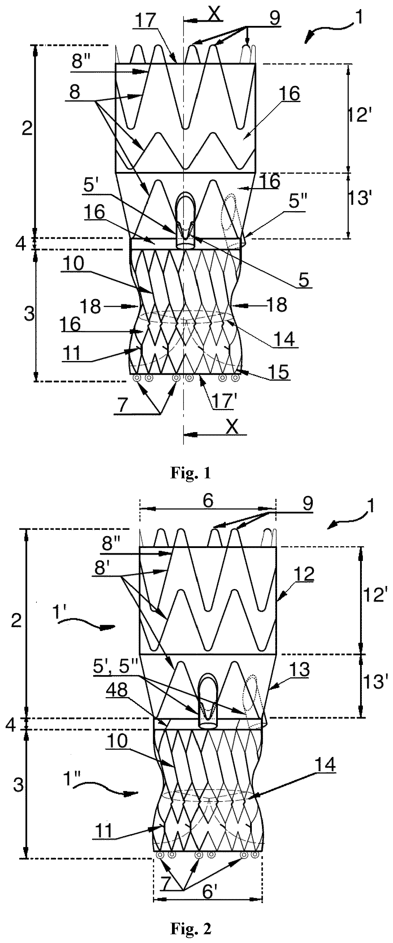

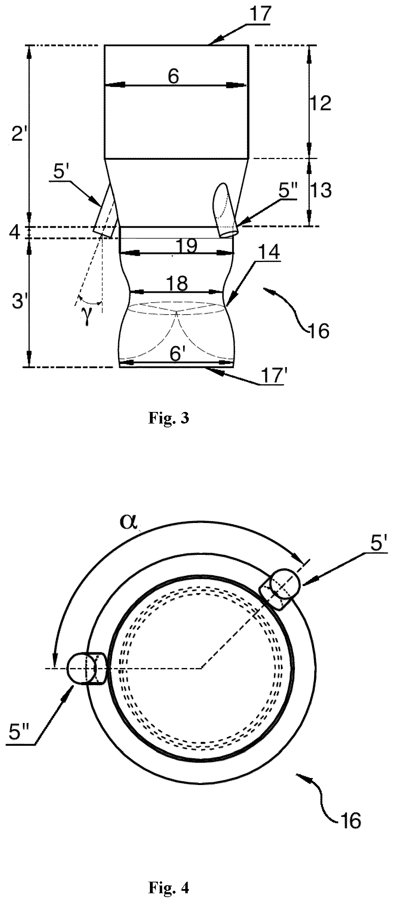

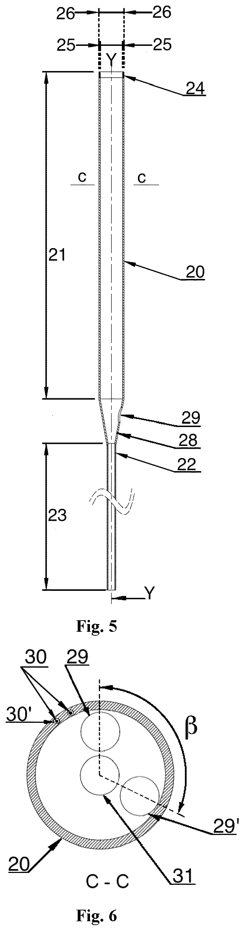

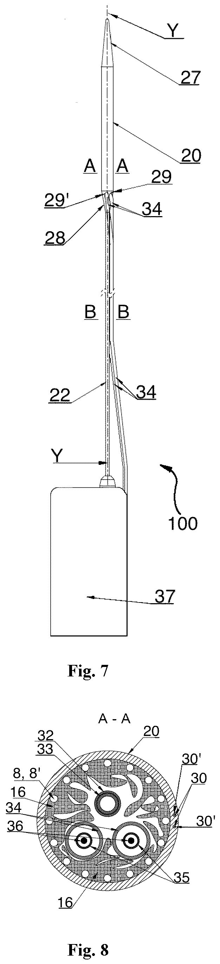

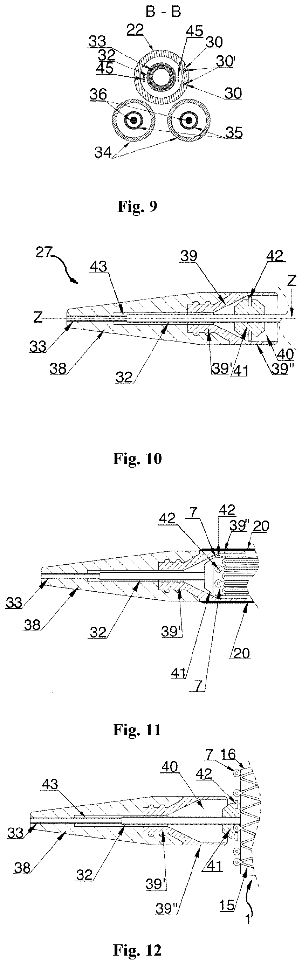

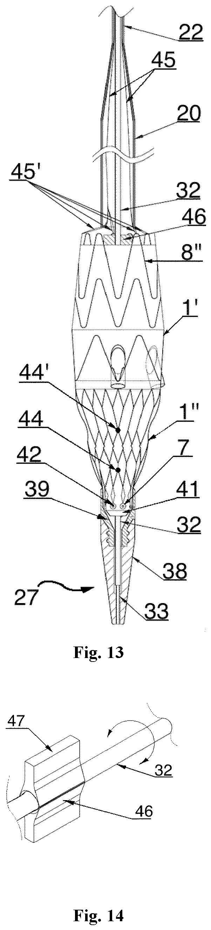

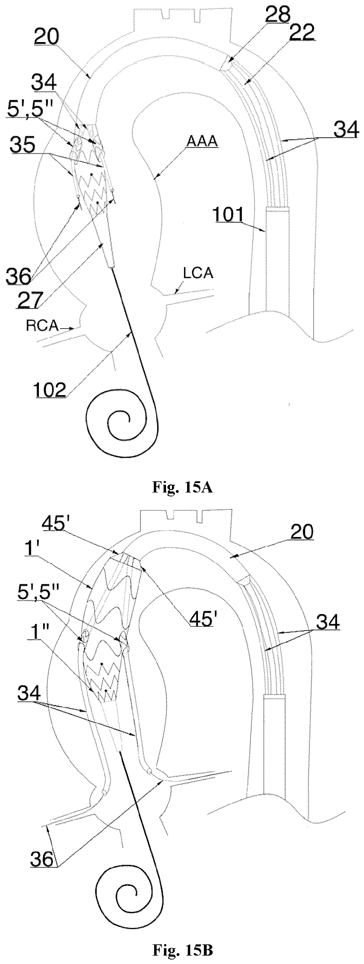

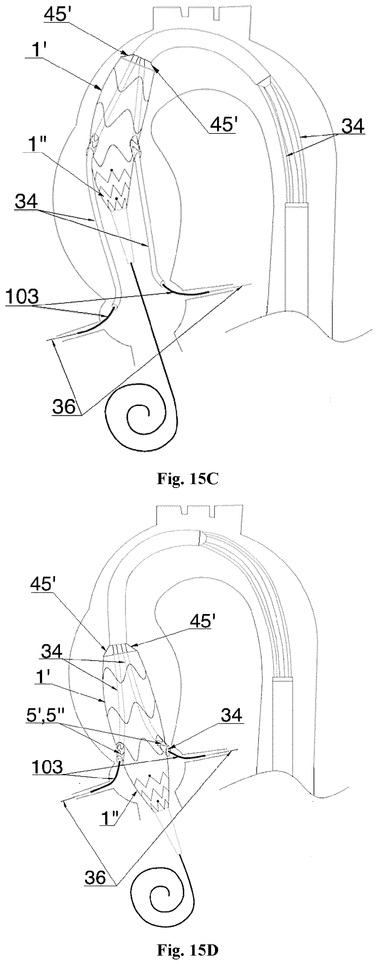

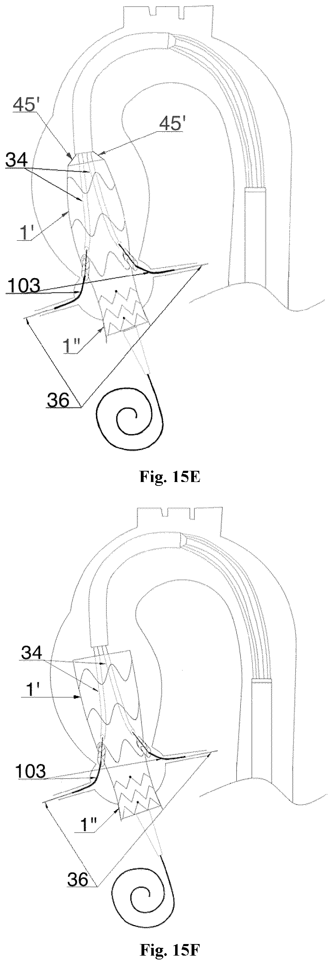

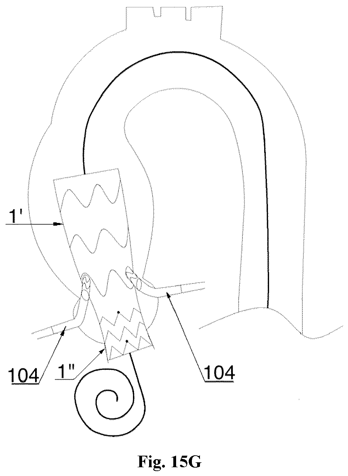

The subject of the invention in an exemplary embodiment is shown in the following drawings where Fig. 1 is a side view of the prosthesis in the first exemplary embodiment, with the first type of the metal frame; Fig. 2 is a side view of the prosthesis in the second exemplary embodiment, with the second type of the metal frame; Fig. 3 is a side view of a coating of the metal frame of the prosthesis; Fig. 4 is an enlarged view of the coating shown in Fig. 3, but presented from the distal side; Fig. 5 is a schematic view of an empty capsule, connected to a catheter shaft, with the cross-section C-C line marked on the figure; Fig. 6 is a schematic C-C cross-sectional view (marked in figure 5) of the capsule, with a view towards its proximal end provided with the catheter shaft (in the center) and two holes for guiding catheters on the sides of the catheter shaft; Fig. 7 is a side view of a transcatheter delivery system comprising the prosthesis in a folded state (here invisible), closed in the capsule, with the cross-section A-A and B-B lines marked on the figure; Fig. 8 is an A-A cross-sectional view (marked in figure 7) of the capsule with the folded prosthesis placed inside; Fig. 9 is a B-B cross-sectional view (marked in figure 7) of the delivery system; Fig. 10 is a schematic view of a system tip, without prosthesis, with receiving means being a part of a distal locking mechanism designed to encapsulate the prosthesis from a distal part of the system; Fig. 11 is a schematic view of the system tip with a folded prosthesis connected, on its distal end, to the receiving means being a part of a distal locking mechanism, wherein the prosthesis is held in the capsule (a distal edge of a capsule can be seen in this figure); Fig. 12 is a schematic view of the system tip with a deployed prosthesis, unlocked, released from the distal locking mechanism, where a metal tube with a locking barrel is brought out of a settling socket; Fig. 13 is a distal portion of the delivery system with a partly deployed prosthesis, which is fully released from the capsule, but still held by the distal locking mechanism placed in the system tip, with a proximal end of the prosthesis being held and positioned by the locking wires of a proximal locking mechanism; Fig. 14 is an enlarged perspective view of a rotating paddle attached to the metal tube above the prosthesis; drawings from Fig. 15A to Fig. 15G show a method of delivering and implanting the prosthesis, where Fig. 15A is a schematic view of the step at which the delivery system is introduced into the ascending aorta and positioned above the coronary arteries level and the capsule is already partially retrieved; Fig. 15B is a schematic view of the step at which the prosthesis is only partly deployed and lateral guide wires are introduced into the right and left coronary arteries; Fig. 15C is a schematic view of the step at which coronary stent graft systems are introduced into the right and left coronary arteries; Fig. 15D is a schematic view of the step at which the delivery system with the prosthesis is moved forward and the stent-valve part, still in locked configuration, is positioned in target location with respect to the native aortic valve; Fig. 15E is a schematic view of the step at which the released, deployed stent-valve part is anchored in target location; 15F is a schematic view of the step at which the stent-graft part is released and fully deployed; Fig. 15G is a schematic view of the step at which implantation of the prosthesis has been completed, coronary stent grafts are in deployed configuration and the system is retracted, but the main guide wire remains as the last one.

### DETAILED DESCRIPTION OF THE INVENTION

The present invention embodies a special type of a vascular prosthesis having a hollow, approximately cylindrical body comprising two interconnected portions, the first one, a stent-valve part comprising an aortic valve fixed inside, the stent-valve part is a distal portion of the prosthesis, and the second one, a stent-graft part provided with at least one branch, the stent-graft part is a proximal portion of the prosthesis. These two parts are joined by a third part of a vascular prosthesis, which is provided between the stent graft part and the stent valve part. Therefore, the mentioned two main parts are combined into one assembly, thus, the prosthesis is an integrated monolithic single medical device.

After deployment, the stent valve part is deposited closer to the heart and the stent graft part is further away from the heart and extends into the ascending aorta near the aortic arch. Along the entire prosthesis, following the course of its longitudinal axis X, a passageway for blood flow is arranged, wherein an inflow is routed through the distal end of the prosthesis and an outflow is routed through the proximal end of the prosthesis.

A transcatheter prosthesis 1, according to the invention, is provided with a self-expanding metal frame 8, 8', 10, on which coating 16 is embedded in such a way that covers at least a portion thereof. The prosthesis comprises a first portion - a stent-valve part 1" of an hourglass shape, provided with an aortic valve 14 placed near the narrowing 18 of the stent valve part 1", and a second portion - a stent-graft part 1' connected with the stent valve part 1" by a cylindrical intermediate part 4 containing either a coating material alone or, in addition, also reinforcing wires (connectors) that link the stent graft part 1' with the stent valve part 1". The intermediate part 4 shows less density of frame wires or is completely devoid of wires and therefore has less stiffness, so its form is not so strictly fixed by shape memory metal elements, making this intermediate part 4 more bendable and susceptible to adapt to the physiological conditions of the tissue where implantation of the prosthesis takes place.

Thus, in the exemplary embodiment of the invention, the prosthesis 1 includes a self-expanding metal frame 8, 8', 10, extending the entire length of the prosthesis 1, the intermediate part 4 is provided with, arranged concentrically around the perimeter of the prosthesis, metal connectors 48 joining the metal frame 8, 8' of the stent graft part 1' with the metal frame 10 of the stent-valve part 1". The prosthesis 1 is equipped with 2 to 10, for example with 2, 3, 4 such connectors 48, which have the shape of straight, oblique, broken, zig-zag or sinuous lines and are more flexible than the rest of the metal frame structure and, as a result, the cylindrical intermediate part 4 is more flexible than the stent graft part 1' and the stent-valve part 1".

In addition, the intermediate part 4 is a shock-absorbing fragment of the prosthesis 1, and thanks to it, for example, movements and vibrations of the stent-valve part 1" are not transferred to the stent-graft part 1', or movements of the stent-graft part 1' (for example, during anchoring of further prostheses to it) are not transferred to the stent-valve part 1".

The metal frame 8, 8' of the stent graft part 1' can also be joined with the metal frame 10 of the stent-valve part 1" by suturing.

In another, preferred embodiment of the invention, presented on Fig. 1, the stent graft part 1' is provided with a self-expanding metal frame 8, 8', and a stent-valve part 1" is provided with its own separate self-expanding metal frame 10. In this embodiment, a cylindrical intermediate part 4, joining the stent graft part 1' and the stent-valve part 1", is prepared from the coating material only and is more flexible than the stent graft part 1' and the stent-valve part 1".

The metal frame 8, 8' of the stent graft part 1', i.e. a first type of the metal frame 8 or a second type of the metal frame 8', in which metal wires are shaped differently and/or arranged differently than in the first type of the metal frame 8, contains rows of wires, which are formed sinusoidally or as zig-zag lines, etc. The metal frame 8, 8', in another design, comprises rows of wires arranged together as a helical structure, but the proximal row of wires 8" is always aligned evenly around the longitudinal axis X of the prosthesis 1. The stent graft part 1' contains a different number of wire rows in different invention embodiments. Various known in the art arrangements can be used, e.g. "peak to peak" or "peak to valley".

The stent graft part 1' comprises, from a proximal side, a cylindrical section 12 terminated on this proximal end by, uncovered by coating 16, peaks 9 of a proximal row of wires 8". On the second side, opposite to the peaks 9, the cylindrical section 12 passes into a tapered (being a part of a cone) section 13 narrowing inwardly towards the cylindrical intermediate part 4. This tapered section 13 is provided with at least one branch 5', 5" (i.e. one or two branches) leading out of orifice located in the tapered section 13. The branches 5', 5" are either directly attached to the aforementioned orifices in the tapered section 13 or through a bellow-shaped intermediate part, which gives the branches more flexibility and freedom to position themselves at different angles with respect to the axis X of the prosthesis 1. The bellow-shaped intermediate part comprises at least one bellows rib, for example from 1 to 6 or from 1 to 4 bellows ribs.

The branch or branches 5', 5" are oriented in relation to each other at an α angle - this is the angle between the longitudinal axes of these branches 5', 5", in a plane perpendicular to the longitudinal X axis of the prosthesis 1. Each of the branches 5, 5', independently, is directed at an acute γ angle, with respect to the longitudinal X axis of the prosthesis 1, toward the stent valve part 1" (as presented in Fig. 3), and the angle γ can range from 5° to 90°, more specifically from 5° to 60° or from 10° to 60, or from 10 to 45°, or from 10° to 30°.

The branch 5', 5" is positioned in proximity of a coronary artery in a deployed state of the prosthesis 1 and is suitable for receiving a coronary stent graft. In the exemplary embodiment of the invention, presented in the Fig. 1, Fig. 2 and Fig. 3, the stent graft part 1' is provided in its tapered section 13 with a first branch 5' and a second branch 5", which have different or the same length and/or diameter. The first branch 5' and the second branch 5" are independently reinforced by branch wires 5, that are configured adjacent to the tapered section 13 or they also surround each branch 5', 5"all around. The term "branch wires" should be understood broadly, including the metal frame achieved by laser cutting process or metal frame braided from wires.

Branches 5', 5" are provided with radiopaque markers, which are embedded at their base, near the body of the stent-graft part 1'.

In the exemplary embodiment of the invention, the stent graft part 1' is almost all the long way covered by the coating 16, and only the peaks 9 of a proximal row of wires 8" are uncovered by coating 16.

Preferably, at least 10% of the length (measured along the longitudinal X axis) of the proximal row of wires 8" is uncovered (called as "free flow" part). In another exemplary embodiment of the invention, at least part of the peaks 9 is uncovered. The uncovered peaks 9 are evenly distributed around the longitudinal X axis of the prosthesis, in several places around the perimeter of the prosthesis 1, at least in two places, such as for example in four, six, or eight places. The uncovered peaks 9 constitute a part of a proximal locking mechanism that holds the prosthesis 1 in the delivery system and provides release and graded manipulation of the prosthesis 1 during the implantation process in the patient's body. All peaks 9 are identical in shape as presented in Fig. 1 and Fig. 2 but they can also have different shape. Preferably, all peaks 9, acting as elements of a proximal locking mechanism, have the same shape and size. The cylindrical section 12 of the stent-graft part 1' remains free in the deployed state, after release of the prosthesis 1 in the ascending aorta, i.e. it is not anchored to the walls of the ascending aorta and, as a result, this cylindrical section 12 constitutes a docking station for attachment of further prosthesis/implants. The proximal row of wires 8" with the uncovered peaks 9 can then provide additional reinforcement to the resulting joint.

The stent-valve part 1" contains the metal frame 10, the design of which is denser than the wireframe of stent-graft part 1', such as rows of struts being more densely arranged, containing additional struts, etc. A person skilled in the art knows different constructions of the self-expanding metal frames of stents, stent-grafts, especially aortic valve assemblies, and all these types can be used herein. A temporary or permanent aortic valve can be used as the stent valve part 1". Valve leaflets can be manufactured from polymers such as thermoplastic polyurethane (TPU), silicone, polyvinyl acetate (PVA), polytetrafluoroethylene (PTFE), polyester (PET), and poly(styrene-block-isobutylene-block-styrene) (SIBS), or from natural tissue like bovine or porcine pericardium, preferably in dry state.

The metal frame 10 of the stent-valve part 1" comprise, on the section between the aortic valve 14 and the distal wire row 15, a plurality of tissue-anchoring barbs 11, placed around a circumference of the metal frame 10 and facing outward from the metal frame 10, set at an acute angle (relative to X axis), preferably some of them towards the proximal end and some others towards the distal end of the prosthesis 1. The aortic valve 14 is preferably mounted above the barbs 11.

On the distal end of the stent valve part", a distal (edge) wire row 15 provided with an uncovered (free of coating 16) distal locking means 7, is placed.

An array of distal locking means 7 is evenly distributed around the longitudinal X axis of the prosthesis 1 and these locking elements have a form of eyelet, hook, or loop, for example, made from the same wire as the metal frame 10. The distal locking means 7 are different in shape, or preferably they are identical, and are placed in at least several places along the prosthesis' 1 circumference, at least at two places, such as two, four, six or eight places. The distal locking means 7 constitutes a part of a distal locking mechanism that holds the distal portion of the prosthesis 1 in the delivery system and provides release of the prosthesis 1 during the implantation process in the patient's body.

The stent-valve part 1", on its metal frame 10, comprises stent valve radiopaque markers 44, 44', in particular marking the position of the aortic valve 14.

In the exemplary embodiment of the invention, the prosthesis 1 comprises a coating 16 along its entire length (except for peaks 9 and distal locking means 7) extending from the coating proximal end 17 to the coating distal end 17' and both branches 5', 5" are covered by coating 16, where the coating 16 may be uniform, of the same material, of equal density over the entire prosthesis 1, or the prosthesis has the first type of coating 16 in the stent-valve part 1" and has the second type of coating 16 in the stent-graft part 1'.

Preferred sizes of the prosthesis 1 are defined below, with lengths given relative to its longitudinal X axis.

The length 2 of the stent graft part 1' is from 20 mm to 80 mm, wherein the coating length 2' of the stent graft part is shorter and ranges from 15 mm to 75 mm. The length of the uncovered peaks 9 section ranges from 3 mm to 50 mm. The length 12' of the coating cylindrical section 12 in the stent graft part 1' is from 10 mm to 65 mm. The length 13' of the coating tapered section 13, in the stent graft part 1', is from 5 mm to 60 mm. Branches 5', 5" have length from 1 mm to 25 mm (defined as a shorter length calculated from their base), preferably from 3 mm or from 5 mm to 25 mm. The intermediate part 4 has length from 1 mm to 10 mm, preferably from 3 to 10 mm.

The stent valve part length 3 is from 15 mm to 45 mm, wherein a coating length 3' of the stent valve part is shorter and ranges from 10 mm to 43 mm. In the exemplary embodiment of the invention, the stent valve part 1' is almost all the long way covered by the coating 16, and only a distal locking means 7 of a distal wire row 15 are free of coating 16 (uncovered). The length of the uncovered distal locking means 7 ranges from 0,5 mm to 10 mm.

The total length of the prosthesis 1 ranges from 35 mm to 125 mm. The proximal end of the prosthesis 1 comprising at least peaks 9 of the proximal row of wires 8" or whole proximal row of wires 8", in deployed state of the prosthesis 1, remains free, i.e. not anchored to the walls of the patient's tissue. This free proximal part of the prosthesis 1 constitutes a docking mechanism for attaching any further stents or prostheses, adapted to the patient's needs.

The proximal diameter 6 of the coating 16 in the prosthesis 1, i.e. the diameter of the proximal end of the coating 16, ranges from 20 mm to 60 mm. The distal diameter 6' (inflow diameter) of the coating 16, i.e. the diameter of the distal end of the coating 16, ranges from 15 mm to 40 mm. The diameter 19 of the proximal section of the stent valve part 1" (outflow diameter) ranges from 15 mm to 50 mm. The proximal diameter 6 is greater than the distal diameter and the diameter 19 of the proximal section of the stent valve part 1".

The diameters given for the coating 16 alone are the same as the diameters of the entire prosthesis 1 at these marked locations, because for the purposes of the invention, the thickness of the coating material or wires in the metal frames are negligible.

The metal wireframe in the prosthesis 1, according to the invention, is formed from a shape memory material, typically from a nickel-titanium alloy (nitinol). The metal frame 8, 8' of the stent-graft part 1' usually is a shaped wire structure, while the metal frame 10 of the stent-valve part 1" is either laser-cut (e.g. from tubing) or has a braided, woven wire construction.

Covering materials forming coating 16, in the prosthesis according to the invention, include expandable polytetrafluoroethylene (ePTFE), polytetrafluoroethylene (PTFE), polyethylene terephthalate (PET), Dacron, polyester, TPU, silicone, etc. Common textile manufacturing techniques, such as weaving, knitting, braiding, and electrospinning, can be used.

The prosthesis 1, according to the invention, is delivered to the target site via the use of a delivery set comprising an introducer comprising an outer sheath and a delivery system 100 configured to deliver the prosthesis 1 to the patient's heart. A self-expanded prosthesis 1 is radially compressed onto the delivery system 100.

Any delivery system 100 can be used to deliver the prosthesis 1, which will ensure the encapsulation of the prosthesis 1 in the crimped position and provides a receiving means cooperating with distal locking means 7 and proximal locking means 9 of the prosthesis 1, which, thanks to them, is maintained partially crimped in length and diameter also after full pull back of the catheter shaft or catheter capsule carrying the prosthesis 1.

The delivery system 100, in the exemplary embodiment, in its innermost space comprises an inner tube 33, extending along a longitudinal Y axis (longitudinal axis of the main body of the delivery system 100 is presented in Fig. 7) from an atraumatic distal tip 27, made by soft polymeric radiopaque material, to a proximal handle 37. The polymeric, preferably multilayer, reinforced and/or braided inner tube 33 is permanently fixed to the tip 27, so the forward and backward movability (distal/proximal direction) of this inner tube 33 provides the same movability of the tip 27. A metal tube 32, with a diameter larger than the diameter of the inner tube 33, is slide-fitted on the inner tube 33. The metal tube 32 is rotatable freely around its longitudinal axis (identical to the Y axis) and relative to the inner tube 33 and also is movable in the distal/proximal direction.

The delivery system 100 comprises an outer tubular catheter shaft 22, having a defined catheter shaft length 23, brought out from the handle 37, wherein in a catheter shaft interior 31 the metal tube 32 and the inner tube 33 are placed. All these three tubular members are arranged concentrically. The catheter shaft 22, on its distal end, is provided with a tubular capsule 20 having a defined length 21 and ending in a radiopaque marker 24 for marking the distal end of the capsule 20. The capsule 20 is connected with the catheter shaft 22 by an intermediate member, a tapered proximal end 28 passing from the smaller diameter of the catheter shaft 22 to the larger, outside diameter of the capsule 26. An inside diameter of the capsule 25 is designed to hold (encapsulate) the compressed, detachably crimped around the metal tube 32, self-expanding prosthesis 1 and safely transport it to the patient's heart. The tapered proximal end 28, located between the capsule 20 and the catheter shaft 22, is provided, on the proximal side, with a first hole 29 for a first guiding catheter 34 and a second hole 29' for a optional second guiding catheter 34. The tapered proximal end 28 is integrated with the capsule 20 and these two segments constitute one element or the tapered proximal end 28 is a separate member connected to the capsule 20.

In the tapered proximal end 28, the first hole 29 and the second hole 29' are oriented in relation to each other at β angle - this is the angle between the longitudinal axes of these first and second holes 29, 29', in a plane perpendicular to the longitudinal Y axis of the capsule 20. The β angle is approximately the same as the α angle described for the positioning of the prosthesis' branches 5', 5", i.e. the defined angles differ by 10% to 20%. In another embodiment of the invention, the β angle ranges from 90° to 180°.

The guiding catheter 34 has construction known in the art and contains inside, along its longitudinal axis, a microcatheter 35 provided in its internal channel with a coronary guide wire 36.

The catheter shaft 22 together with the capsule 20 is configured to slide between a distal-most position over the inner tube 33 and the metal tube 32, in which the capsule 20 has closed position, and a proximally retracted configuration, in which the capsule has open position, in which the prosthesis 1 restrained in it is released.

In the tubular wall of the catheter shaft 22 and the capsule 20, at least one steering wire channel 30' provided with steering wire 30 is routed, along the entire length of the catheter shaft 22 and capsule 20. As presented in Fig. 6, Fig. 8 and Fig. 9, in the exemplary embodiment of the invention, the delivery system 100 preferably comprises the first and the second steering wire channels 30' provided with the first and the second steering wires 30 (pull wires). The steering wires 30 can steer the distal section of the catheter shaft 22 with the capsule 20, providing deflection of this section and more accurate positioning of the capsule 20 in the docking position, especially relative to the aortic axis.

The tip 27 of the delivery system 100 comprises: an atraumatic distal part 38; the inner tube 33 routed from it along the longitudinal Z axis of the tip 27; and at some distance from the distal part 38, a metal tube channel 43 with a metal tube 32 inside it; and also comprises a proximal part 39 that is permanently connected to the distal part 38 through the fixing end 39'. The proximal part 39 is terminated on its proximal side (capsule side) by a sleeve part 39" containing a settling socket 40 inside, and the diameter of the sleeve part 39" is the same as the diameter of the capsule 20. The settling socked 40 together with the interior of the capsule 20 forms the tubular cavity designed to carry the folded prosthesis 1 and contain elements of a distal locking mechanism to secure the prosthesis from the distal side, and elements of a proximal locking mechanism to secure the prosthesis from the proximal side. The settling socked 40 and the capsule 20 have together close state, when the prosthesis 1 is encapsulated and open state for releasing the prosthesis 1, with the opening being gradual (in stages).

The distal locking mechanism, contained in the delivery system 100, is provided with a permanently concentrically embedded on a flexible (preferably with laser cutting patterns) metal tube 32, on its distal portion, at least partially circular barrel 41, provided on its outer surface, on its circular part, with distal receiving elements 42, preferably in a form of at least two protruding tabs, studs or pins. The receiving elements 42 are part of the locking mechanism cooperating with the distal locking means 7 disposed at the distal (edge) wire row 15 of the prosthesis 1. The receiving elements 42 and the barrel 41 together constitute the receiving means 41, 42 of the distal locking mechanism.

In another embodiment of the invention, the receiving elements 42 and locking means 7 are shaped differently, i.e. the distal locking means 7 are in a form of pins or barbs, facing inside the prosthesis, and the barrel 41 contains receiving elements 42 in a form of holes or a concave groove routed around the circumference of the barrel 41 (around the longitudinal Y/Z axis). It is clear to the person skilled in the art that to ensure this cooperation, the sizes of the receiving elements 42 and of the locking means 7 are matched to each other.

The barrel 41 is disposed on the metal tube 31 at some distance from the distal end of the metal tube 31 placed in the distal part of the metal tube channel 43, which is longer than the metal tube 31. The tip 27 is provided with a portion of empty metal channel 43, so the metal tube 31 together with the barrel 41 mounted on it, is movable in a distal/proximal direction and, as a result, the position of the barrel 41 and the locked prosthesis 1 in the settling socket 40 (being the locking chamber) is controlled. The metal tube 32 together with barrel 41 is also rotatable freely around its longitudinal axis (identical to the Y axis) and relative to the inner tube 33.

The proximal locking mechanism, contained in the delivery system 100, includes at least one inelastic locking wire 45 (presented in Fig. 13) brought out of the handle 37 and routed, as a one piece of wire, along the longitudinal axis Y of the delivery system 100, up to the peak 9 or peaks 9 of the proximal row of wires 8" of the prosthesis 1. The locking wire 45, i.e. wire for stent graft part releasing, forms at its distal end, a locking wire loop 46, which is threaded through the peak 9 and returns to the handle. Each locking wire 45 begins and ends in the handle. The proximal locking mechanism comprises from 2 to 8 locking wires 45, i.e. from 2 to 8 locking loops 45'. As presented in Fig. 13, the delivery system 100 comprises four locking wires 45 bent into four locking loops 46 conducted through four peaks 9 and, in this example, there are 8 ends of the locking wires 45 (two for each wire) on the handle 37.The proximal locking mechanism is gradual, i.e. loosening the locking wires 45 graduates the opening of the prosthesis 1. The locking loops 45' have several degrees of relaxation (elongation) providing successive phases of prosthesis 1 opening.

The proximal locking mechanism with locking wires 45 also ensures efficient, fast and precise placement of the prosthesis in the delivery capsule 20. The locking wires 45 have a maximally stretched position and the prosthesis 1 then has a folded position with the greatest length and smallest diameter, which allows the prosthesis 1 to be inserted into the capsule. This position of the locking wires 45 keeps the prosthesis 1 in folded state when the capsule 20 is retracted.

The locking wires 45 have also a partially relaxed position, allowing the prosthesis to be partially opened. As long as the locking wires 45 are connected to the peaks 9, it is possible to correct the state of the prosthesis 1. In the end of the unlocking stage, the locking wires 45 are completely removed, one by one, to achieve full prosthesis 1 release.

Therefore, two independent prosthesis 1 release arrangements are provided: (i) gradual retraction of the capsule 20 and gradual exposure of the prosthesis 1, and (ii) gradual reduction of the stretching force of the locking wires 45 and, in the next stage, removal of the wires 45 for at first partial and finally full release of the prosthesis 1.

The delivery system is also provided with a rotating paddle 46 (shown in enlarged view in Fig. 14, where its movement is marked by arrows), which is positioned inside the capsule 20 and durable mounted to the metal tube 32, on the section where the proximal row of wires 8" is placed. The folded prosthesis 1 is crimped onto the metal tube 32 and locked between the barrel 41 and the rotating paddle 46 as presented in Fig. 13. The barrel 41 and the rotating paddle 46 rotate along with the metal tube 32, on which they are mounted and, as a result, the rotational movement of the whole prosthesis 1 is ensured. The rotation can be performed when the prosthesis 1 is in configuration, when the peaks 9 of the proximal row of wires 8" of the prosthesis 1 are still inside the capsule.

Fig. 13 illustrates a slightly open prosthesis 1, partially exposing the rotating paddle 46 (the rest of the rotating paddle 46 is covered by the proximal edge of the prosthesis 1), but, in fact, the prosthesis 1 at the transport and positioning stage is clamped tighter so that the rotating paddle 46 catches the uncovered proximal wire row 8" to achieve rotation of the entire prosthesis.

Fig. 1 shows the longitudinal X-axis of prosthesis 1, Fig. 5 and Fig. 6 show the longitudinal Y-axis of the capsule 20 and delivery system 100, and fig. 10 shows the longitudinal Z-axis of the tip 27, but in the delivery system 100 containing the folded prosthesis 1 the aforementioned axes are consistent with each other, as the position of the aforementioned members is coaxial.

The steps undertaken to deploy the prosthesis 1, using the exemplary delivery system as defined above, are described below. Initially, before the delivery system 100 is used, an introducer of the appropriate size, adapted to the size of the delivery system 100, is inserted into the blood vessel of the patient. The procedure for inserting the introducer is generally known.

Firstly, with reference to Fig. 15A, the main guide wire 102 is percutaneously inserted into the ascending aorta such that it extends through the aorta and the delivery system 100 with the prosthesis 1, held in a contracted state by a capsule 20, is introduced over the main guide wire 102 into the aorta, but above the left and right coronary arteries. The term "above" should be understood as presented on the drawings. The delivery system 100 is inserted using the introducer 101. When the delivery system 100 is inserted and the capsule 20 is closed, at least the lateral guide wire 36 or two lateral guide wires 36 are already in a ready position, i.e. guided through the one branch 5', 5" or two branches 5', 5" of the prosthesis 1 surrounded by the constricting capsule 20, so the operator does not have to insert these lateral guide wires 36 into the coronary vessels through the entire delivery system 100 but only slide them out of the branches 5', 5". Optionally, also at least one microcathater 35, preferably two microcathaters 35 and/or at least one guiding catheter 34, preferably two guiding catheters 34, are in a ready position, i.e. guided through the branches 5', 5" of the prosthesis 1.

Next, as shown in Fig. 15A, the capsule 20 is retracted at least above the distal end of the branches 5', 5", preferably up to the partially deployed configuration, as defined above. The lateral guide wire(-s) 36, along with microcatheter(-s) 35 and guiding catheter(-s) 34, preloaded in the delivery system 100, are exposed. In this partially folded position, it is possible to rotate the entire prosthesis 1 by rotating the metal tube 32 assembly comprising the permanently joined barrel 41 and rotating paddle 46. This is shown in detail in Fig. 13, where the distal locking means 7 are placed on the receiving elements 42, in the form of pins, mounted on the barrel 41, and the so obtained joints were locked in the settling socket 40 acting as locking chamber. At the same time the proximal peaks 9 of prosthesis 1 are held in a partially closed position (by locking wires 45), so that this part adheres around the rotating paddle 46. In this way, the physician rotates the metal tube 32 to rotate the prosthesis 1 and determine the proper position of the branches 5', 5" in relation to the coronary arteries.

Next, as presented in Fig. 15B, the lateral guide wire(-s) 36 are introduced into the right and left coronary arteries (or only one guide wire 36 is used and introduced into the left or right artery). The physician can also either (optionally) use a microcatheters 35 (or only one microcatheter 35) inserted in the coronary arteries over the lateral guide wires 36 and then insert guiding catheters 34 (or only one guiding catheter 34) into these arteries or, omitting the microcatheters 35, and directly insert guiding catheter(-s) 34 into the arteries over the lateral guide wire(-s) 36. Next, as shown in Fig. 15C, the coronary stent graft system(-s) 103 carrying stent graft(-s) 104 are inserted, through the inside of the guiding catheter(-s) 34, over the lateral guide wire(-s) 36, into the coronary arteries. If microcatheter(-s) 35 have been used they are withdrawn and coronary stent graft system(-s) 103 are inserted in their place, over the lateral guide wire(-s) 36. At this stage, the delivery system 100 is still in the same previous position - the distal tip 27 is positioned above the coronary arteries.

Next, as shown on Fig. 15D, the delivery system 100 with the prosthesis 1 is moved forward and the stent-valve part 1", still not fully deployed, in locked configuration, is pushed over the main guide wire 102 and on the guiding catheter 34 or two guiding catheter 34 up to the target site - the aortic valve, where the stent-valve part 1" will align with the location of the native aortic valve.

Then, the stent-valve part 1" is unlocked which results in its release, deployment and anchorage in the tissue surrounding the native valve as presented in Fig. 15E. The release of the stent-valve part 1" occurs so that the physician pushes forward the distal tip 27, exposing the distal edge of the prosthesis 1 and barrel 41, so that the distal locking means 7, for example in the form of eyelets (shown on Fig. 1, Fig. 2, Fig. 13), discharge from the settling socket 40 due to a self-expanding force of the metal frame 10.

Next, as presented in 15F, the stent-graft part 1' is released and fully deployed by pulling out the locking wires 45 from the proximal locking means in the form of peaks 9. Physician can gradually loosen the locking wires 45 and gradually deploy the proximal part of the prosthesis 1. It is also possible that physician will only pull-out part of the locking wires 45 achieving a stepwise release of the stent-graft part 1'. Gentle and gradual unfolding of the stent prevents transmission of movement/vibration to the already fixed stent-valve part 1".

Next, after full deployment of the prosthesis 1, the coronary stent-graft(-s) 104, provided by the coronary stent graft system(-s) 103, are deposited in the left and right coronary arteries, as shown on Fig. 15G, wherein these stent-graft(-s) 104 are connected to the branches 5', 5" by overlapping so that the resulting joint is at least 1 mm long or 2 mm long. After that step, the whole delivery system 100 and the lateral guide wire(-s) 36 and the guiding catheter(-s) 34 are retracted. The main guide wire 100 is removed last.

The described method can be implemented with a prosthesis 1 provided with one branch 5', 5" and only one lateral guide wire 36, one microcatheter 35 and one guiding catheter 34 can be used. In another implementation of the invention, the prosthesis 1 has two branches 5', 5"and two guide wires 36, two microcatheters 35 and two guiding catheters 34 are used accordingly.

The prosthesis according to the invention is implanted in the patient's beating heart during the methods with transfemoral access, transiliac access or transcarotid access. A transfemoral procedure is shown as an example on Fig. 15A to Fig. 15G, but it will be obvious to the physician to adapt the described method to be carried out through other vessels, i.e. through the carotid artery, for example. The transcarotid procedure may be less complicated, as it is not necessary to guide the delivery catheter (system) through the aortic arch. Then the delivery system/catheter does not need to be provided with the steering wires.

### Designations in the drawings:

1 - prosthesis
1' - stent-graft part
1" - stent-valve part
2 - length of the stent-graft part
2' - coating material length in the stent-graft part
3 - length of the stent-valve part
3' - coating material length in the stent-valve part
4 - intermediate part (joining part)
5 - branch wire
5' - first branch
5" - second branch
6 - proximal diameter
6' - distal diameter
7 - distal locking means: eyelet, loop, hook
8 - first type of the metal frame
8' - second type of the metal frame
8" - proximal (edge) wire row
9 - peak (of the proximal wire row)
10 - metal frame of the stent-valve part
11 - barb
12 - cylindrical section
12' - length of the cylindrical section coating (in the stent graft part)
13 - tapered section
13' - length of the tapered section coating (in the stent graft part)
14 - aortic valve
15 - distal (edge) wire row
16 - coating
17 - proximal end of coating
17' - distal end of coating
18 - narrowing
19 - diameter of the proximal section of the stent valve part
20 - capsule
21 - capsule length
22 - catheter shaft
23 - catheter shaft length
24 - radiopaque marker
25 - inside diameter of the capsule
26 - outside diameter of the capsule
27 - tip
28 - tapered proximal end of capsule
29 - first hole
29' - second hole
30 - steering wire
30' - steering wire channel
31 - interior of the catheter shaft
32 - metal tube
33 - inner tube
34 - guiding catheter
35 - microcatheter
36 - lateral guide wires
37 - handle
38 - distal part of the tip
39 - proximal part of the tip
40 - settling socket
41 - barrel
42 - receiving element: tab, stud, pin
39' - fixing end
39"- sleeve part
43 - metal tube channel
44, 44' - markers (of the stent valve part)
45 - locking wire
45' - locking loop
46 - rotating paddle
47 - rotating paddle edge
48 - connector
100 - delivery system
101 - introducer
102 - main guide wire
103 - coronary stent graft system
104 - stent graft
AAA - ascending aorta aneurysm
RCA - right coronary artery
LCA - left coronary artery
X - implant longitudinal axis
Y - capsule and catheter shaft longitudinal axis
Z - tip longitudinal axis
α - angle between branches
β - angle between the first hole (for the first guiding catheter) and the second hole (for the second guiding catheter)
γ - angle of inclination of the branches with respect to the longitudinal X axis

## Claims

1. A self-expanding, covered at least partially by a coating (16), endoluminal prosthesis, comprising:
- as a distal portion, a stent-valve part (1"), containing a metal frame (10) and an aortic valve (14) secured inside it, the metal frame (10) is provided, with distal locking means (7), evenly distributed around the perimeter of the prosthesis (1), at its distal edge,
- as a proximal portion, a stent-graft part (1'), containing a metal frame (8, 8') provided, at its proximal edge, with proximal locking means (9), evenly distributed around the perimeter of the prosthesis (1),
- an intermediate part (4), joining the stent-valve part (1") with the stent-graft part (1') and having less stiffness than the stent-valve part (1") and the stent-graft part (1'), the intermediate part (4) contains either a coating (16) alone, or, in addition, also metal connectors (48) linking the metal frame (10) of the stent-valve part (1") with the metal frame (8, 8') of the stent-graft part (1'),
wherein the stent graft part (1') comprises, from a proximal side, a cylindrical section (12) passing into a tapered section (13) narrowing inwardly towards the intermediate part (4), the tapered section (13) is equipped with at least one branch (5', 5") covered by the coating (16), and wherein the distal locking means (7) and the proximal locking means (9) are not covered by the coating (16).

2. A self-expanding prosthesis according to claim 1, wherein the proximal locking means (9) have the form of peaks (9) of a proximal row of wires (8").

3. A self-expanding prosthesis according to claim 1, wherein the distal locking means (7) are in the form of eyelets, hooks or loops.

4. A self-expanding prosthesis according to claim 1, wherein the coating (16) covers entire prosthesis (1) and only the distal locking means (7) and the proximal locking means (9) remains uncovered by the coating (16).

5. A self-expanding prosthesis according to claim 1 wherein the branch (5', 5") or branches (5', 5") is/are directed at an acute γ angle, with respect to the longitudinal X axis of the prosthesis (1), toward the stent valve part (1"), and the angle γ range from 5° to 60°.

6. A self-expanding prosthesis according to claim 1, wherein the branch (5', 5") or branches (5', 5") are attached to the tapered section (13) through a bellow-shaped intermediate part.

7. A delivery system comprising the prosthesis (1) according to claim 1.

8. A delivery system according to claim 7, wherein it comprises:
- an inner tube (33), extending along a longitudinal Y axis of the delivery system (100), from an atraumatic distal tip (27) to a handle (37);
- a metal tube (32), slide-fitted on the inner tube (33), and extending from the internal space of the distal tip (27) to a handle (37);
- an outer tubular catheter shaft (22) slide-fitted on the metal tube (32), the catheter shaft (22) is brought out from the handle (37) and is terminated by a capsule (20), containing inside the radially compressed on the metal tube (32) prosthesis (1);
- the capsule (20) has closed position when is closed by the tip (27) and has open position when the capsule (20) and the tip (27) are displaced relative to each other;
wherein the distal part of the metal tube (32) is equipped with receiving means (41, 42) on which the distal locking means (7) of prosthesis (1) are removably embedded and blocked in the inner space of the tip (27) and the delivery system (100) comprises also removable holding means (45, 45') retaining the proximal locking means (9) of the prosthesis (1), so that when the capsule (20) is in its fully open position, the prosthesis (1) is still maintained partially compressed, holding its crimping length.

9. A delivery system according to claim 7 or 8, wherein it comprises preloaded, guided through the branch (5', 5") or branches (5', 5") of the prosthesis (1), at least one lateral guide wire (36) routed in the proximal part of the delivery system (100) along the internal channel of the microcatheter (35) and of the guiding catheter (34).

10. A delivery system according to claim 7 or 8 or 9, wherein on the metal tube (32) a rotating paddle (46) is permanently mounted in the section where the stent-graft part (1') of the prosthesis (1) is placed.

11. A delivery system according to claim 7 or 8 or 9 or 10, wherein the receiving means (41, 42) are in the form of pins (42) mounted on a barrel (41).

12. A delivery system according to any one of claims 7 to 11, wherein the holding means (45, 45') are in the form of locking loops (45') of locking wires (45).

13. A method of delivering the endoluminal prosthesis defined in claim 1 to the patient's ascending aorta and aortic valve, using the delivery set comprising an introducer and a delivery system containing the endoluminal self-expanding prosthesis, the method comprising:
m) inserting the introducer into the blood vessel;
n) inserting a main guide wire into the blood vessel such that it extends through the blood vessel into the ascending aorta and aortic valve;
o) moving forward, over the main guide wire, preferably using the steering wire(-s) when crossing the aorta, and positioning up to above the level of the coronary arteries the compressed, deployable, locked from the proximal side and from the distal side, prosthesis, located inside the constricting capsule of the delivery system;
p) retrieving the constricting capsule up to the partially released configuration;
q) rotating the prosthesis to get alignment at least between one prosthesis branch and one coronary ostium;
r) introducing the at least one lateral guide wire, preloaded in the delivery system by positioning it/them in the branch or branches of the prosthesis, into the coronary artery (-ies), using the guiding catheter(-s), or optionally also the microcatheter(-s), as support for coronary(-ies) cannulation;
s) introducing, over the lateral guide wire(-s), the coronary stent graft system(-s) carrying coronary stent graft(-s), into the coronary artery(-ies);
t) moving forward the inner part of the delivery system comprising the inner tube and a metal tube with the prosthesis, over the main guide wire and preferably over the at least one guiding catheter up to the aortic valve, where the stent-valve part of the prosthesis will align with the proper location with respect to the native aortic valve;
u) unlocking the distal side of the prosthesis releasing its stent-valve part which anchors in the tissue surrounding the native valve;
v) unlocking the proximal side of the prosthesis releasing its stent-graft part;
w) depositing, after full deployment of the prosthesis, the coronary stent-graft(-s), provided by the coronary stent graft system(-s), in the left and/or right coronary arteries, wherein the expanded stent-graft(-s) are connected to the branch(-es) of the prosthesis by overlapping;
x) retracting the coronary stent graft system(-s), guiding catheter(-s) and the lateral guide wire(-s), the delivery system, and finally also the main guide wire,
wherein during the stages from (b) to (h) a positioning of the prosthesis is carried out, by its rotation, around its longitudinal axis, together with the metal tube, on which it is removably mounted, and wherein during the stages from (d) to (g) the delivery system is approximately still in the same position where the distal tip is positioned above the level of the coronary arteries.

14. A method according to claim 13, wherein in stage (h) unlocking the distal side of the prosthesis and releasing its stent-valve part is performed according to the on/off mode, and unlocking the proximal side of the prosthesis and releasing its stent-graft part in stage (i) is performed gradually.
